Europäisches Patentamt

European Patent Office

Office européen des brevets

(19)

(11) Veröffentlichungsnummer : **0 123 016**
**B1**

(12) **EUROPÄISCHE PATENTSCHRIFT**

(45) Veröffentlichungstag der Patentschrift :
**16.06.87**

(51) Int. Cl.⁴ : **A 61 F   2/38**

(21) Anmeldenummer : **84100430.2**

(22) Anmeldetag : **17.01.84**

(54) **Gelenkendoprothese für ein Kniegelenk.**

(30) Priorität : **23.03.83 CH 1594/83**

(43) Veröffentlichungstag der Anmeldung :
**31.10.84 Patentblatt 84/44**

(45) Bekanntmachung des Hinweises auf die Patenterteilung : **16.06.87 Patentblatt 87/25**

(84) Benannte Vertragsstaaten :
**AT DE FR GB IT NL SE**

(56) Entgegenhaltungen :
**FR-A- 2 136 541**
**FR-A- 2 417 971**
**GB-A- 2 021 419**

(73) Patentinhaber : **GEBRÜDER SULZER AKTIENGESELL-
SCHAFT**
**Zürcherstrasse 9**
**CH-8401 Winterthur (CH)**

(72) Erfinder : **Horber, Willi**
**Turbinenstrasse 12**
**CH-8005 Zürich (CH)**

(74) Vertreter : **Dipl.-Ing. H. Marsch Dipl.-Ing. K. Sparing
Dipl.-Phys.Dr. W.H. Röhl Patentanwälte
Rethelstrasse 123
D-4000 Düsseldorf (DE)**

## Beschreibung

Die Erfindung betrifft eine Gelenkendoprothese für ein Kniegelenk, bestehend aus einem Femurteil mit zwei Kondylenschalen, die vorderseitig mindestens durch einen Steg und rückseitig durch eine Verbindungswand miteinander verbunden sind, wobei zwischen Steg und Verbindungswand in einem mittleren Bereich eine in Streckstellung des Gelenkes im wesentlichen senkrecht zur Richtung der Streckstellung verlaufende Ueberbrückung vorhanden ist, weiterhin bestehend aus einem Tibiateil, bei dem beiderseits eines, zwischen die Kondylenschalen des Femurteils ragenden, geneigte Flächen aufweisenden Zapfens Auflage- und Gleitflächen für die Kondylenschalen vorgesehen sind, wobei die Verbindungswand des Femurteils den Zapfen des Tibiateils vorderseitig und seitlich umgibt und wobei ferner die Oberfläche des Zapfens an seiner Basis mit Spiel den distalen seitlichen Bereichen der Verbindungswand des Femurteils gegenübersteht.

Der vorstehend geschilderte, konstruktive Aufbau einer Kniegelenk-Prothese ist aus der GB-A-2 021 419 bekannt ; eine ähnliche Konstruktion zeigt die FR-A-2 417 971. Das wesentliche Merkmal dieser — und ähnlicher — Konstruktionen besteht darin, dass ein Beugen des Gelenks mindestens im wesentlichen bei wandernder Drehachse durch gleitendes Abrollen der Kondylenschalen des Femurteils auf den Auflagerflächen des Tibiateils erfolgt. Die Erfindung bezieht sich also mit anderen Worten auf die Gruppe der Kniegelenk-Prothesen, die frei von scharnier- oder kugelgelenkartigen Fixierungen des Drehpunktes sind.

Eine Rotation des Unterschenkels und Fusses um die Längsachse eines Beines erfolgt bekanntlich bei gestrecktem Knie im Hüftgelenk, während diese Drehung im Kniegelenk ausgeführt wird, sobald dieses aus der Streckstellung auch nur leicht abgebeugt worden ist. Aufgabe der Erfindung ist es, diese Rotationsbewegung bei Kniegelenk-Prothesen der beschriebenen Art nicht nur zu ermöglichen, sondern darüberhinaus zu führen ; die Auflage bei der und für die Führung der Bewegung soll möglichst gross sein, um eine Verteilung der dabei auftretenden Kräfte zu erreichen. Bei den diskutierten Konstruktionen ist eine Führung der « Längsrotation » gar nicht oder nur unvollständig möglich, wobei eine Führung wenn überhaupt nur längs einer Linie erfolgt.

Die geschilderte Aufgabe wird erfindungsgemäss dadurch gelöst, dass die Oberfläche des Zapfens im Basisbereich in Richtung von der Vorderseite zur Rückseite konvex gekrümmte, gegen die Zapfenachse geneigte Führungsflächen bildet und daß die distalen seitlichen Bereiche der Verbindungswand des Femurteiles an die Führungsflächen angepasste Schrägflächen bilden, die unter dem gleichen Winkel zur Zapfenachse wie die Führungsflächen verlaufen.

Die angestrebte Führung der Rotationsbewegung erfolgt bei der erfindungsgemässen Prothese durch Gleiten oder Abrollen der Schrägflächen des Femurteils auf den Führungsflächen des aus dem Tibiateil herausragenden Zapfens ; die gleiche und konstante Neigung beider Flächen gegen die Vertikale bzw. die Zapfenachse gewährleistet eine relativ breite Auflage von Führungs- und Schrägfläche aufeinander, wodurch zusätzlich eine Führung gewährleistet ist, wenn der Unterschenkel relativ zum Oberschenkel um einige Grad abgekippt ist (Varus-valgus-Bewegung). Da mindestens während des grössten Teils einer Beugebewegung des Knies ein Teilbereich der Kondylenschalen des Femurteils auf den Auflageflächen des Tibiateils aufleigt, bleibt auch immer die Führung eines Teilbereichs der Schrägflächen an den Führungsflächen bei einer Rotation um die Längsachse des Unterschenkels gewährleistet.

Zweckmässigerweise werden die seitlichen Führungsflächen in ventral-dorsaler Richtung so geformt, dass eine geführte Rotation in einem Winkelbereich von ± 20° möglich ist.

Bei fehlenden oder — z. B. in Beugestellung — schlaffen Seitenbändern ist ein Abkippen des Unterschenkels gegenüber dem Oberschenkel in Richtung medial/lateral oder eine Varus-valgus-Bewegung möglich. Um bei diesem Abkippen ein Klemmen des Zapfens an den seitlichen Bereich der Rückwand zu verhindern, kann es vorteilhaft sein, wenn die Zapfenoberfläche des Tibiateils oberhalb des Basisbereichs eine die Neigung dieses Bereichs übersteigende Neigung gegen die Zapfenachse hat. Die « oberen » Zapfenbereiche sollten dabei so geformt sein, dass sie Varus-valgus-Bewegungen von ± 10° ermöglichen.

Im folgenden wird die Erfindung anhand eines Ausführungsbeispiels im Zusammenhang mit der Zeichnung näher erläutert.

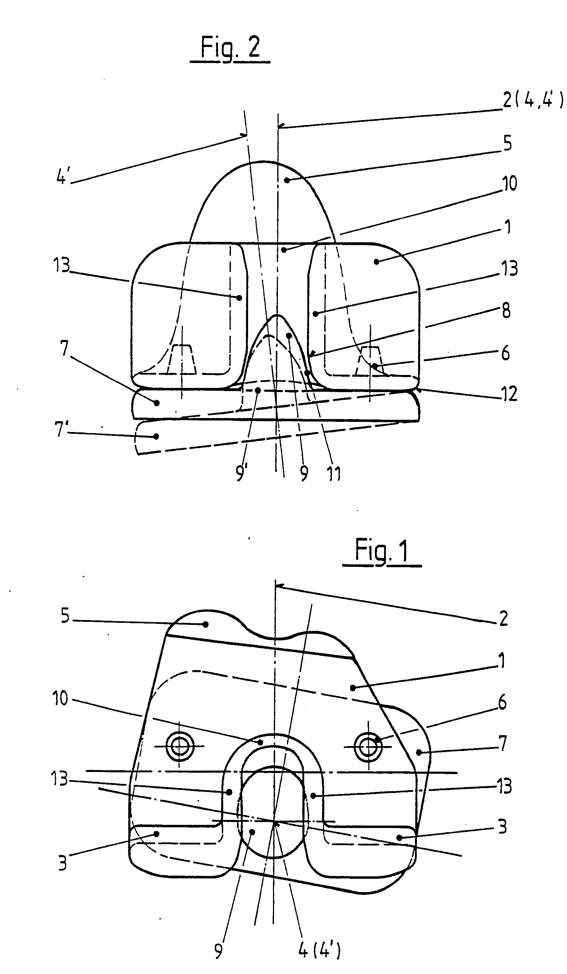

Figur 1 zeigt in einer Aufsicht in Richtung der Femurlängsachse schematisch eine Ausführungsform des neuen Kniegelenks, während.

Figur 1 eine Ansicht des gleichen Gelenkes von dorsal nach ventral gesehen wiedergibt.

Der am distalen Ende eines nicht gezeigten Femurknochens zu fixierende Femurteil 1 (Fig. 1) hat beidseitig einer Sagittalebene 2 (Fig. 2) je eine an ihrer Aussenseite kondylenartig ausgebildete Schale 3, die bei einer Bewegung des Kniegelenks auf einem Tibiateil 7 gleitend abrollt und dabei im wesentlichen die Belastungskräfte überträgt. Die Flächennormale zur in Streckstellung des Gelenkes im wesentlichen horizontalen Tangentialebene an die Kondylenflächen der Schalen 3 fällt in die Sagittalebene 2 ; sie ist mit 4 bezeichnet. Auf der in Fig. 1 nach oben gerichteten Vorderseite des Gelenkes sind die Kondylenschalen 3 über einen Patella-Schild 5 als Steg miteinander verbunden. Bezüglich der Sagittalebene 2 ist der Patella-Schild 5 derart unsymmetrisch ausgebildet, dass sein Flächenanteil lateral, d. h. zur Aussenseite hin, grösser ist als nach medial. Die Fig. 1 und 2 zeigen somit einen für einen linken Femurknochen bestimmten Femurteil 1.

Der Patella-Schild 5 geht über eine Ueberbrückung, die dem Knochen zugewandt Verankerungszapfen 6 trägt, in eine Verbindungswand über, die die — in Fig. 1 nach unten gerichteten — dorsalen Bereiche der Kondylenschalen 3 untereinander verbindet. Diese Verbindungswand 10 besteht aus einer in Richtung der Flächennormalen 4 (Fig. 2) aufrecht stehenden Hohlzylinderschale, deren konvexe Aussenseite dem Knochen zugewandt ist. Die Schale ist auf der dem Knochen abgewandten Aussenseite der Prothese über gerade seitliche Bereiche 13 mit den dorsalen Abschnitten der Kondylenschalen 3 verbunden.

Erfindungsgemäss bilden die distalen Enden der seitlichen Bereiche 13 der Verbindungswand 10 nach oben aufeinander zulaufende Schrägflächen 8 (Fig. 2). Diesen Schrägflächen 8 gegenüberliegend sind im Basisbereich eines Zapfens 9, der zwischen den Auflageflächen des Tibiateils 7 nach oben ragt, in Richtung von der Vorderseite zur Rückseite konvex gekrümmte, gegen die Zapfenachse geneigte Führungsflächen 11 vorgesehen, wobei zwischen beiden Flächen 8 und 11 ein Spiel von ca. 2 mm vorhanden ist ; in Streckstellung des Gelenkes fällt die Zapfenachse 4' mit der Flächennormalen 4 zusammen, während sie sich beim Beugen des Knies ohne eine Varusvalgus-Bewegung in der Sagittalebene 2 bewegt.

Bei einer Rotation des abgewinkelten Gelenkes werden die Schrägflächen 8 des Femurteils 1 an den Führungsflächen 11 des Zapfens geführt. Die Neigungen der an die Führungsflächen 11 angepaßten Schrägflächen 8 und der Führungsflächen 11 gegen die Sagittalebene 2 sind gleich. Der Querschnitt des Zapfens 9 kann kreis- oder ellipsenförmig oder oval ausgebildet sein ; er muss dabei lediglich so stark gekrümmt sein, dass er in beiden Richtungen, d. h. nach links oder nach rechts relative Verdrehungen des Femurs gegen die Tibia um die Flächennormale 4 als Drehachse von je 20° erlaubt.

Oberhalb der Führungsflächen 11 vergrössert sich die Neigung oder Krümmung des Zapfens 9 gegen die Sagittalebene 2. Dadurch wird eine Varus-valgus-Bewegung des Unterschenkels gegen den Oberschenkel, d. h. ein Abkippen des Tibiateils 7, gegenüber den Femurteil 1 — beispielsweise um eine Achse 12 in eine mit 7' bezeichnete Schräglage (Fig. 2) — ohne ein Klemmen des Zapfens 9 in den seitlichen Bereichen 13 der Verbindungswand 10 gewährleistet. Der Zapfen 9 sollte dabei in seinem Oberteil so geformt sein, dass dieses Abkippen 10° betragen kann, ohne dass Schwierigkeiten auftreten.

## Patentansprüche

1. Gelenkendoprothese für ein Kniegelenk, bestehend aus einem Femurteil (1) mit zwei Kondylenschalen (3), die vorderseitig mindestens durch einen Steg (5) und rückseitig durch eine Verbindungswand (10) miteinander verbunden sind, wobei zwischen Steg (5) und Verbindungswand (10) in einem mittleren Bereich eine in Streckstellung des Gelenkes im wesentlichen senkrecht zur Richtung der Streckstellung verlaufende Ueberbrückung vorhanden ist, weiterhin bestehend aus einem Tibiateil (7), bei dem beiderseits eines, zwischen die Kondylenschalen (3) des Femurteils (1) ragenden, geneigte Flächen aufweisenden Zapfens (9) Auflage- und Gleitflächen für die Kondylenschalen (3) vorgesehen sind, wobei die Verbindungswand (10) des Femurteils (1) den Zapfen (9) des Tibiateils (7) vorderseitig und seitlich umgibt und wobei ferner die Oberfläche des Zapfens (9) an seiner Basis mit Spiel den distalen seitlichen Bereichen (13) der Verbindungswand (10) des Femurteils (1) gegenübersteht, dadurch gekennzeichnet, dass die Oberfläche des Zapfens (9) im Basisbereich in Richtung von der Vorderseite zur Rückseite konvex gekrümmte, gegen die Zapfenachse (4') geneigte Führungsflächen (11) bildet und daß die distalen seitlichen Bereiche (13) der Verbindungswand (10) des Femurteiles (1) an die Führungsflächen (11) angepasste Schrägflächen (8) bilden, die unter dem gleichen Winkel zur Zapfenachse (4') wie die Führungsflächen (11) verlaufen.

2. Gelenkendoprothese nach Anspruch 1, dadurch gekennzeichnet, dass die Zapfenoberfläche des Tibiateils (7) oberhalb des Basisbereichs eine die Neigung dieses Bereichs übersteigende Neigung gegen die Zapfenachse (4') hat.

## Claims

1. A knee joint endoprosthesis comprising a femur part (1) having two condyl shells (3) interconnected frontally at least by a web (5) and rearwardly by a connecting wall (19), a bridge which extends substantially perpendicularly to the direction of the extended position of the joint when the same is in such position being provided between the web (5) and the wall (10), the prosthesis also comprising a tibia part (7) in which on either side of a pin (9) which extends between the condyl shells (3) and which has inclined surfaces there are bearing and rubbing surfaces for the condyl shells (3), the connecting wall (10) of the femur part (1) extending frontally and laterally around the pin (9) of the tibia part (7), the surface of the pin (9) being disposed at its base, and with clearance, opposite the distal lateral zones (13) of the connecting wall (10) of the femur part (1), characterised in that the surface of the pin (9) forms in the base zone guide surfaces (11) which are convex from the frontal to the rear side and which are inclined to the pin axis (4'), and the distal lateral zones (13) of the wall (10) form inclined surfaces (8) which are adapted to the guide surfaces (11) and which extend at the same angle to the pin axis (4') as the guide surfaces (11).

2. An endoprosthesis according to claim 1, characterised in that the pin surface of the tibia part (7) has above the base zone such an inclination to the pin axis (4') as exceeds the inclination of such base zone.

**Revendications**

1. Endoprothèse articulée pour articulation rotulienne, comprenant une partie fémorale (1) présentant deux calottes condyliennes (3) reliées l'une à l'autre à la face antérieure au moins par l'intermédiaire d'une membrure (5) et à la face postérieure par l'intermédiaire d'une paroi de jonction (10), un pont s'étendant sensiblement perpendiculairement à la direction de la position étirée, lorsque l'articulation occupe cette position étirée, se trouvant dans une région médiane entre la membrure (5) et la paroi de jonction (10), ladite prothèse comprenant en outre une partie tibiale (7) dans laquelle des surfaces de contact et de glissement pour les calottes condyliennes (3) sont prévues de part et d'autre d'un tenon (9) qui possède des surfaces inclinées et s'étend entre les calottes condyliennes (3) de la partie fémorale (1), la paroi de jonction (10) de la partie fémorale (1) entourant, à la face antérieure et latéralement le tenon (9) de la partie tibiale (7), la surface du tenon (9) faisant par ailleurs face avec jeu, à sa base, aux régions latérales distales (13) de la paroi de jonction (10) de la partie fémorale (1), caractérisée par le fait que la surface du tenon (9) forme, dans la zone de base, des surfaces de guidage (11) qui accusent une courbure convexe de la face antérieure à la face postérieure et sont inclinées par rapport à l'axe (4') du tenon ; et par le fait que les régions latérales distales (13) de la paroi de jonction (10) de la partie fémorale (1) forment des surfaces inclinées (8) qui sont adaptées aux surfaces de guidage (11) et s'étendent, par rapport à l'axe (4') du tenon, selon le même angle que les surfaces de guidage (11).

2. Endoprothèse articulée selon la revendication 1, caractérisée par le fait que la surface du tenon de la partie tibiale (7) présente par rapport à l'axe (4') du tenon, au-dessus de la zone de base, une inclinaison plus forte que l'inclinaison de cette zone.

## Fig. 2

## Fig. 1